# EUROPEAN PATENT APPLICATION

(11) **EP 4 588 427 A1**
(43) Date of publication of application: **23.07.2025**
(21) Application number: 23864659.0
(22) Date of filing: 11.09.2023
(51) Int. Cl.: A61B 5/00, A61B 18/12, A61B 1/005, A61M 25/00, A61M 31/00, A61B 1/04, A61B 1/06

(54) **MEDICAL CATHETER**

(30) Priority: 15.09.2022 CN 202211123870
(71) Applicant: Shanghai MicroPort Medical (Group) Co., Ltd., Shanghai 201203 (CN)
(72) Inventor: ZHOU, Qi, Shanghai 201203 (CN); ZHANG, Zhenghai, Shanghai 201203 (CN); YUE, Bin, Shanghai 201203 (CN)
(74) Representative: Lavoix
(86) International application number: PCT/CN2023/117991
(87) International publication number: WO 2024/055927

(57) **Abstract**

The present invention relates to a medical catheter including a catheter body and a steering component. The catheter body includes a proximal portion and a distal portion at a distal end of the catheter body. The distal portion is configured for image-based monitoring and for release of therapeutic energy and/or a therapeutic agent. The distal portion is connected to the steering component and configured to bend relative to the proximal portion under the control of the steering component. When used in a therapeutic procedure, the catheter of the invention can provide real-time image-based monitoring of therapeutic efficacy and adjust a bending angle of the distal portion at any time, as desired, to bring the distal portion into close contact or abutment of a lesion. This enables accurate targeting of the lesion in need of therapeutic treatment, resulting in improved therapeutic outcomes and a higher success rate of the surgical procedure.

## Description

### TECHNICAL FIELD

The present invention relates to the field of medical devices and, in particular, to a steerable medical catheter with both diagnostic and therapeutic capabilities.

### BACKGROUND

Atherosclerosis, a syndrome that affects arterial blood vessels, develops as a chronic inflammatory response in arterial walls, in large part due to plaques in arterial walls resulting from deposits of lipids, macrophages and foam cells. Atherosclerosis is commonly referred to arteriosclerosis, and its pathophysiology manifests as several types of lesions, ranging from fibrous, to lipidic, to calcified. In the current clinical practice, established therapeutic options for atherosclerosis mainly include medication, intervention and bypass surgery. However, all these approaches are associated with a number of problems including restenosis and thrombosis over time.

Thermo-physiotherapy has been widely used in clinical practice thanks to relatively low cost, few side effects and short treatment time. Thermo-physiotherapy can be accomplished by thermal ablation, and available energy source options for thermal ablation primarily include cryoballoons, focused ultrasound waves, laser light and radio frequency (RF) radiation. Clinical studies have shown that RF ablation can provide a range of advantages, including determined safe frequencies, controllable thermal energy output and ease of functional integration. In order to ensure good thermo-physiotherapeutic performance, ablation-induced tissue damage or destruction must be limited in a determined range. However, at present, techniques remain absent for accurate ablation range monitoring and control for fibrous plaques.

Intravascular imaging is a mainstream monitoring technique, which majorly involves optical coherence tomography (OCT), intravascular ultrasound (IVUS), angioscopy, intravascular magnetic resonance imaging (MRI), etc. Compared with other imaging techniques, OCT is profoundly superior in resolution (higher than 10 µm) and can be used to capture high-definition images of biological tissues, which are very helpful in accurate imaging and identification of plaques within blood vessels. However, in therapeutic procedures, diagnosis is typically separate from therapeutic treatment, necessitating frequent exchange between imaging and ablation catheters. This associates such treatment with higher overall cost, absence of immediate availability and, in some circumstances, a remaining high rate of recurrence. Further, such therapeutic procedures often lack efficacy or even fail due to inadequate contact or abutment of an ablation catheter with tissue (lesion).

### SUMMARY

It is an objective of the present invention to provide a medical catheter, which overcomes problems that may arise from the use of conventional therapeutic treatment, including unsatisfactory efficacy or even failure due to inadequate contact or abutment of a catheter with tissue of interest, as well as those caused by separate diagnosis.

To this end, the present invention provides a medical catheter, which includes a catheter body and a steering component. The catheter body includes a proximal portion and a distal portion at a distal end of the catheter body. The distal portion is configured for image-based monitoring and for release of therapeutic energy and/or a therapeutic agent. The distal portion is connected to the steering component, and is configured to bend relative to the proximal portion under the control of the steering component.

In one embodiment, the distal portion may include a distal tubular body, and the proximal portion may include a proximal tubular body, wherein hardness of the distal tubular body is less than hardness of the proximal tubular body.

In one embodiment, the steering component may include a steering traction element and a steering member, the steering member disposed at a proximal end of the catheter body, the steering traction element distally connected to the distal portion, a proximal end of the steering traction element passed through the catheter body and then connected to the steering member, the steering traction element configured to be driven by the steering member to control the bending of the distal portion.

In one embodiment, a plurality of steering traction elements may be provided, which are equidistantly arranged circumferentially around a central axis of the catheter body.

In one embodiment, each of the steering traction elements may be connected to at least different one of steering members.

In one embodiment, the catheter may further include an interface portion, wherein a proximal end of the proximal portion is connected to the interface portion, and the steering member is movably disposed on the interface portion.

In one embodiment, the catheter body may further include a steering channel extending along an axis thereof, wherein the steering traction element is passed through the steering channel.

In one embodiment, the steering component may include a magnetically responsive deformable component and/or a photo-deformable component, the magnetically responsive deformable component made of a magnetically responsive material and deformable under the action of a magnetic field, the photo-deformable component made of a photo-responsive material and photo-deformable when absorbing light energy.

In one embodiment, the catheter may further include an interface portion, wherein a proximal end of the proximal portion is connected to the interface portion, and the interface portion is configured for connection with an external device and for input and output of information.

In one embodiment, the distal portion may include an image-based diagnostic member and a therapeutic member, the image-based diagnostic member configured for the image-based monitoring, the therapeutic member configured for the release of the therapeutic energy and/or the therapeutic agent,
the image-based diagnostic member including an imaging probe, wherein the catheter body further includes an imaging channel extending along an axis thereof, in which an imaging transmission structure connected to the imaging probe is disposed, and a central axis of which coincides with the central axis of the catheter body.

In one embodiment, the distal portion may further include an imaging window, wherein the imaging probe is provided at the imaging window.

In one embodiment, the imaging probe may be an optical probe, wherein the imaging transmission structure includes an imaging optical fiber, a protective tube and a torsion spring, the protective tube disposed over the imaging optical fiber, the torsion spring disposed between the protective tube and the imaging optical fiber, one end of the imaging optical fiber connected to the imaging probe, the other end of the imaging optical fiber extending through the imaging channel to a proximal end of the catheter body.

In one embodiment, the catheter may further include an interface portion, wherein a proximal end of the proximal portion is connected to the interface portion; the interface portion includes an imaging interface and a mechanical-power transmission interface; the other end of the imaging optical fiber is connected to the mechanical-power transmission interface and the imaging interface; and the image-based diagnostic member is configured to be driven by a driving means to rotate circumferentially and/or translate axially with respect to the catheter body.

In one embodiment, the therapeutic member may include an energy output means capable of outputting at least one of radio frequency (RF) radiation, ultrasound waves, laser light and a cryofluid as the therapeutic energy, and/or a therapeutic agent output means including at least one of a drug-containing coating and a drug-releasing structure, the drug-releasing structure including a drug-delivery reservoir and/or drug-delivery microneedles.

In one embodiment, the therapeutic member may include the energy output means, wherein the energy output means includes an electrode, and the distal portion further includes a temperature-measuring element disposed on the electrode and configured to measure a surface temperature of the electrode.

In one embodiment, the catheter body may further include a temperature-control fluid channel extending along the axis thereof, wherein the distal portion further includes temperature-control fluid output holes provided in the electrode, and the temperature-control fluid channel is distally connected to the temperature-control fluid output holes and configured for transfer of a temperature-control fluid.

In one embodiment, the catheter body may further include a temperature-control lead channel and an electrode lead channel, both extending along the axis thereof, wherein the temperature-measuring element is connected to a distal end of a temperature-control lead; a proximal end of the temperature-control lead extends through the temperature-control lead channel to the proximal end of the catheter body; the electrode is connected to a distal end of an electrode lead; and a proximal end of the electrode lead extends through the electrode lead channel to the proximal end of the catheter body.

In one embodiment, the energy output means may include a plurality of electrodes for outputting RF radiation, which are spaced axially along and/or circumferentially around the catheter body.

In one embodiment, the distal portion may be distally connected to a tip through an elastic coupling portion, the tip defining a guidewire lumen.

Compared with the prior art, the proposed catheter offers at least the benefits as follows:
The distal portion in the catheter is configured both for image-based monitoring and for release of therapeutic energy and/or a therapeutic agent, and therefore can provide both diagnosis and therapeutic treatment. Additionally, it is bendable under the control of the steering component. With this arrangement, interventional treatment of a lesion site can be carried out without possibly frequent exchange between an imaging catheter and a therapeutic catheter, which may require relocating the site, thereby simplifying the surgical procedure and ensuring accurate locating. In addition, during the therapeutic procedure, the distal portion can monitor therapeutic efficacy in real time, helping in accurate control of therapeutic energy and/or therapeutic agent output. Coupled with the steering capabilities of the steering component, which allow the bending angle of the distal portion to be adjusted at any time, as required, it is ensured that the distal portion is always maintained in desirable contact or abutment with the lesion, achieving accurate therapeutic targeting of the lesion, improving therapeutic outcomes, resulting in an increase in the success rate of the procedure and reducing the time that the procedure requires. Apart from these, the steering capabilities make the catheter suitable for use in a wider range of applications with more flexibility and convenience for therapeutic treatment of lesions of various shapes. Therefore, the proposed catheter can be used for accurate therapeutic treatment of lesions with simplified surgeon's operation. Further, the catheter can be repeatedly used in a therapeutic procedure until all the identified lesions are therapeutically treated, resulting in material savings, helping reduce the incidence of complications that conventional interventional surgery often suffers from, improving clinical outcomes, lowering the rate of re-hospitalization, and relieving household burdens and social and economic losses incurred by diseases. Therefore, it is beneficial in terms of both economy and ecology.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 is a schematic diagram showing the overall structure of a medical catheter according to an embodiment of the present invention;
Fig. 2 is a schematic diagram showing the structure of a medical catheter according to an embodiment of the present invention in detail;
Fig. 3 is a schematic structural view of a medical catheter according to a first embodiment of the present invention, showing a steering component therein in detail;
Fig. 4 is an enlarged view of portion a of the interventional catheter of Fig. 3;
Fig. 5a is a cross-sectional view of the structure of Fig. 4 taken along line A-A;
Fig. 5b is a cross-sectional view of the structure of Fig. 4 taken along line B-B; and
Fig. 5c is a cross-sectional view of the structure of Fig. 4 taken along line C-C.
Fig. 6 shows an application of a medical catheter according to an embodiment of the present invention, in which the catheter is delivered into a blood vessel over a guidewire running through a guidewire lumen in a tip; and
Fig. 7 shows an application of a medical catheter according to an embodiment of the present invention, in which a distal portion is bent relative to a proximal portion into contact or abutment with a lesion (fibrous plaque).

### List of Reference Numerals

10 medical catheter; 1 interface portion; 11 fluidic interface; 12 imaging interface; 13 electrical interface; 14 mechanical-power transmission interface; 2 catheter body; 21 imaging channel; 22 temperature-control fluid channel; 23 temperature-control lead channel; 24 electrode lead channel; 25 steering channel; 210 proximal portion; 220 distal portion; 221 therapeutic member; 222 image-based diagnostic member; 2221 imaging probe; 2222 imaging transmission structure; 223 imaging window; 224 temperature-control fluid output hole; 225 proximal electrode; 226 distal electrode; 3 tip; 31 guidewire lumen; 4 steering component; 41 steering traction element; 42 steering member; 5 coupling portion; 6 temperature-control lead; 7 electrode lead; 20 fibrous plaque; 30 guidewire.

### DETAILED DESCRIPTION

The present invention will be described in greater detail below with reference to the accompanying drawings, which present preferred embodiments thereof. It will be understood that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of an actual implementation are described in this specification. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art. The present invention is described in greater detail in the following paragraphs with reference to the accompanying drawings. From the following description, advantages and features of the invention will become more apparent. Note that the figures are provided in a very simplified form not necessarily drawn to exact scale for the only purpose of helping in describing the embodiments in a convenient and clear way.

In addition, the terminology used herein is for the purpose of describing particular embodiments only and is not intended to be limiting of this application. As used herein, the singular forms "a", "an" and "the" are intended to include the plural forms as well, unless the context clearly indicates otherwise. It will be understood that, as used herein, the terms "a", "an" and the like do not denote a limitation of quantity, but rather denote the presence of at least one of the referenced items. "Plurality" means two or more than two. Unless defined otherwise herein, the terms "distal", "proximal", "upper", "lower" and/or the like are merely for ease of description, and should not be construed as being limited to a particular position, or to a particular spatial orientation. The term "including", "comprising" or the like is meant to encompass the elements or items listed thereafter and equivalents thereof but do not preclude the presence of other elements or items. It will also be understood that, as used herein, the term "a number of" refers to an uncertain quantity.

In the following, for ease of illustration, the terms "distal", "proximal", "axial" and "circumferential" may be used. When used to describe a medical catheter, "distal" refers to an end away from an operator who is operating the catheter, while "proximal" refers to an end closer to the operator. "Axial" refers to a direction defined along a central axis of the catheter, and "circumferential" refers to a direction defined about the central axis. The central axis refers to a lengthwise direction of the catheter. "Radial" refers to a direction defined along a diameter of the catheter.

The present invention will be further described below with reference to the accompanying drawings, which illustrate preferred embodiments thereof. If there is no conflict, the embodiments described hereunder and features thereof can complement or be combined with each other.

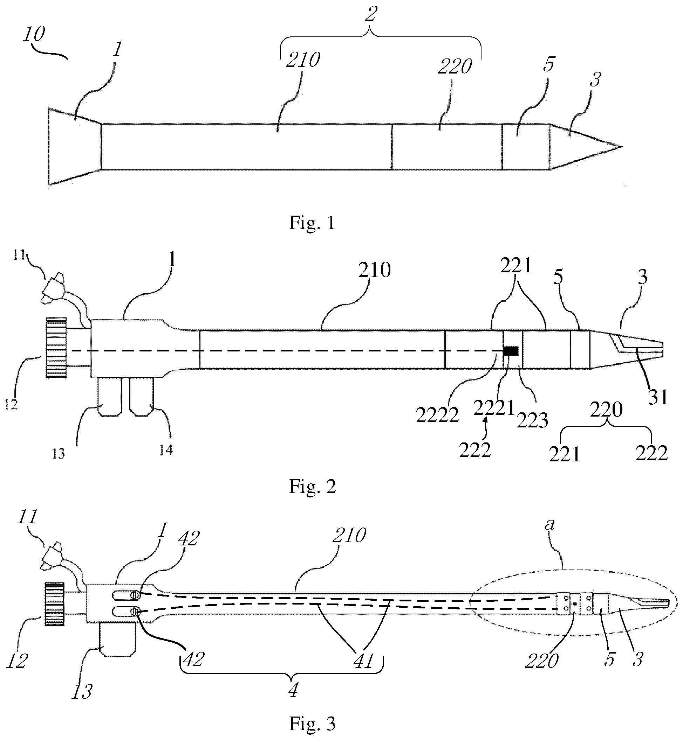

As shown in Figs. 1 and 2, in an embodiment of the present invention, there is provided a medical catheter 10, the medical catheter 10 has both diagnostic and therapeutic capabilities, and the distal end of the medical catheter 10 has the bendability. The medical catheter 10 of this embodiment is not limited to being use for vascular intervention, but can also be used for interventional treatment of non-vascular lumens, such as esophageal, prostatic and intestinal. Preferably, the medical catheter 10 of this embodiment is used for coronary intervention, such as interventional treatment of atherosclerosis. In these applications, good therapeutic results can be expected.

Specifically, the medical catheter 10 includes a catheter body 2. The catheter body 2 includes a proximal portion 210 and a distal portion 220 at a distal end of the catheter body 2. The distal portion 220 is capable of image-based monitoring of a tubular lumen of interest and release of therapeutic energy and/or a therapeutic agent to a target site in the tubular lumen of interest. The tubular lumen of interest may be a vascular or non-vascular lumen. The present invention is not limited to any particular type of therapeutic energy. For example, it may be at least one of radio frequency (RF) radiation, ultrasound waves, laser light and a cryofluid. The therapeutic agent is essentially a drug. As shown in Fig. 3, the medical catheter 10 of this embodiment further includes a steering component 4. The steering component 4 is disposed at least partially within the catheter body 2. The distal portion 220 is connected to the steering component 4 and configured to bend relative to the proximal portion 210 under the control of the steering component 4.

Thus, the medical catheter 10 of the present invention can function as both an imaging catheter and a therapeutic catheter. Through combining diagnostic and therapeutic capabilities in a single catheter, interventional treatment of a lesion can be carried out without exchange between an imaging catheter and a therapeutic catheter, which may require relocating a site in need of therapeutic treatment, thereby simplifying the surgical procedure and making it easier to carry on. Moreover, the distal portion 220 can monitor a therapeutic effect of the procedure in real time, enabling accurate targeting of a lesion in need of therapeutic treatment and making the procedure more accurate and effective. Further, during the procedure, the steering component 4 can be used at any time, as needed, to make adjustments in a bending angle of the distal portion 220 such that the distal portion 220 always remains in close contact or abutment with a lesion being treated. This enables accurate alignment with the lesion and hence accurate targeting thereof, which can result in significant improvements in therapeutic outcomes and a higher success rate of the surgical procedure.

The present application is not limited to any particular value of the bending angle of the distal portion 220. For example, it may be less than or equal to 90°, or greater than 90° and less than 180°. The bending angle of the distal portion 220 may be properly configured in practical applications.

The distal portion 220 may employ one or more imaging techniques to provide image-based monitoring of the tubular lumen of interest, such as at least one of optical imaging and ultrasonic imaging, preferably optical coherence tomography (OCT), which provides the highest resolution among the currently available imaging techniques, helping in imaging and identification of intravascular plaques.

The distal portion 220 is able to release one or more types of therapeutic energy, such as at least one of RF radiation, ultrasound waves, laser light and a cryofluid.

The distal portion 220 may employ one or more mechanisms to deliver a therapeutic agent to the target site. The therapeutic agent is preferred to be a drug. The present invention is not limited to any particular type of drug, and any desired drug is possible, such as anti-proliferative, anti-hyperplastic, anti-restenotic, anti-inflammatory, antibacterial, anti-tumor, anti-mitotic, anti-metastatic, anti-thrombotic, anti-osteoporotic, anti-angiogenic, cytostatic or microtubule-inhibitory.

In one embodiment, the distal portion 220 includes a therapeutic member 221 and an image-based diagnostic member 222. The therapeutic member 221 is configured to release at least one of therapeutic energy and a therapeutic agent to the target site (with one or more lesions, such as fibrous plaques). The therapeutic member 221 is able to output at least one of RF radiation, ultrasound waves, laser light and a cryofluid as the therapeutic energy, and/or the therapeutic member 221 is able to release the therapeutic agent to the target site using one or more mechanisms. The image-based diagnostic member 222 is configured to capture images of a diseased region, which are helpful in pre-operative identification of locations and compositions of lesions, intra-operative monitoring of a therapeutic effect or release of the therapeutic agent, and post-operative scanning-based evaluation of therapeutic outcomes after the therapeutic treatment of the diseased region is completed. This application is not limited to any imaging method that the image-based diagnostic member 222 employs. The image-based diagnostic member 222 may adopt optical or ultrasonic imaging, preferably optical coherence tomography (OCT) imaging.

In the present embodiment, the distal portion 220 further includes a distal tubular body, and the proximal portion 210 includes a proximal tubular body. The therapeutic member 221 is disposed on the distal tubular body, and the image-based diagnostic member 222 is received in the distal tubular body. For example, the therapeutic member 221 may include electrodes disposed on an outer circumferential surface of the distal tubular body. As another example, the therapeutic member 221 includes a drug-delivery reservoir provided in the outer circumferential surface of the distal tubular body and extending through a wall thereof. As a further example, the therapeutic member 221 includes a drug-containing coating covering the outer circumferential surface of the distal tubular body. It will be understood that the distal tubular body may itself define the therapeutic member 221 in the form of, for example, a drug-delivery reservoir, drug-delivery microneedles, etc. That is, the therapeutic member 221 is integrally formed with the distal tubular body. Alternatively, the therapeutic member 221 is arranged on the distal tubular body as a separate component in the form of, for example, electrodes, a drug-containing coating, etc.

It will be understood that the proximal and distal tubular bodies may be separately manufactured and then assembled with each other. Alternatively, they may be integrally formed, without needing the subsequent assembly. In order to impart increased steerability, hardness of the distal tubular body is preferably less than that of the proximal tubular body. Accordingly, the distal tubular body is overall flexible and hence more easily steerable, and the proximal tubular body is overall stiff and easier to advance and manipulate. A proximal end of the distal tubular body may be joined to a distal end of the proximal tubular body, or integrally formed therewith. In a specific example, the proximal and distal tubular bodies are both made of a polyamide material, such as nylon, PEBAX, etc., and the proximal tubular body may be provided as a multi-layer tube including an inner layer, an outer layer and at least one metal layer (e.g., in the form of a braided tube or spiral coil) between the inner and outer layers. With this arrangement, the proximal portion 210 has increased strength or stiffness. However, in alternative embodiments of the present application, proximal and distal tubular bodies may be made of materials with different degrees of hardness.

The present application is not limited to any particular structure of the steering component 4. As shown in Fig. 3, in one embodiment, the steering component 4 includes a steering traction element 41 and a steering member 42. The steering member 42 is disposed at a proximal end of the catheter body 2. The distal end of the steering traction element 41 is coupled to the distal portion 220, and the proximal end of the steering traction element 41 is passed through the catheter body 2 and then coupled to the steering member 42. The steering traction element 41 is configured to be driven by the steering member 42 to control bending of the distal portion 220.

There may be one or more, typically a plurality of, steering traction elements 41. In the latter case, there may be two or more steering traction elements 41. In principle, the number of steering traction elements 41 is even, such as, two, four, six or more. Preferably, the steering traction elements 41 are even arranged circumferentially around a central axis of the catheter body 2, enabling bending in any desired direction and facilitating 360° bending. In this way, more flexible and more easy steering can be achieved, making the catheter suitable for use in a wider range of applications. Typically, four or six steering traction elements 41 are provided, which can satisfy the diagnostic and therapeutic requirements of most practical applications. Preferably, the steering traction elements 41 are made of a metal material with a long service life, good resilience, high accuracy after repeated flexing and high yield strength, such as a nickel-titanium alloy, stainless steel, etc. The steering traction elements 41 may be in any suitable form, such as pull cords, wires or rod-shaped elements. Preferably, the steering traction elements 41 are wires, such as round wires, optionally with a diameter of 0.01 mm to 0.2 mm. Such a diameter can ensure that the wires will not be easily broken while not increasing the overall size of the catheter.

The steering traction elements 41 may be controlled by individual steering members 42. The number of steering members 42 may be equal to that of steering traction elements 41, or not. For example, each steering traction element 41 may be controlled by a single steering member 42, or each steering traction element 41 may be controlled by more of the steering members 42. In one embodiment of the present application, the steering traction elements 41 are coupled to respective steering members 42 in a one-to-one correspondence. That is, each steering traction element 41 is controlled by a respective one of the steering members 42. In this way, a simple, easy-to-implement structure can be provided, which allows more accurate, more reliable, easier steering operation. The steering members 42 may be steering knobs or steering slider, which can be rotated or moved to loosen or tighten the steering traction elements 41.

As shown in Figs. 1 and 2, in one embodiment, the medical catheter 10 further includes an interface portion 1, and the proximal end of the proximal portion 210 is coupled to the interface portion 1. The steering members 42 are movably arranged on the interface portion 1. Here, by "movably arranged", it is intended to mean that the steering members 42 are arranged on the interface portion 1 in such a manner that they can be rotated or translated relative to the interface portion 1. In alternative embodiments of the present application, the interface portion 1 may be omitted.

In an alternative embodiment, the steering component 4 may be a magnetically responsive deformable component disposed within the distal tubular body, which is made of a magnetically responsive material. This material bends under the action of an external magnetic field, causing the distal portion 220 to bend relative to the proximal portion 210. When the magnetic field disappears, the magnetically responsive deformable component recovers its original shape, causing the distal portion 220 to also recover its original shape. More specifically, the magnetically responsive deformable component may be made of a polymer material with magnetically responsive particles (e.g., Fe₃O₄, NdFeB, etc.) dispersed therein and shaped like a rod. It may be disposed around the middle of the distal tubular body, but is not limited to being located at any particular axial position. The magnetically responsive deformable component can deform in various directions at different degrees under the action of an external magnetic field, causing the distal portion 220 to bend at any desired angle.

In an alternative embodiment, the steering component 4 may be a photo-deformable component disposed within the distal tubular body, which is made of a photo-responsive material and bends when absorbing light energy, causing the distal portion 220 to bend relative to the proximal portion 210. For example, the photo-deformable component may be a strip-shaped photosensitive element attached to an inner wall of the distal tubular body, which is deformable when exposed to light, causing the distal portion 220 to bend relative to the proximal portion 210. Moreover, when no longer exposed to light, the photo-deformable component recovers its original shape, causing the distal portion 220 to also recover its original shape. More specifically, the photo-deformable component may be provided as a strip-shaped structure made of a photo-deformable material (e.g., an azobenzene polymer, or PLZT ceramic), which is attached to the inner wall of the distal tubular body. A lighting component in the medical catheter 10 may illuminate and thereby deform a specified portion of the photo-deformable component, causing the distal portion 220 to bend at a desired angle.

Preferably, the steering component 4 for bending the distal portion 220 assumes a form with a small footprint. It should be noted that the steering component 4 may employ one or more mechanisms to deliver the steering function. Compared with the other mechanisms, the steering traction elements 41 are simpler in structure and easier to implement technically. In addition, they have a smaller footprint, helping in control of the overall size of the medical catheter 10.

According to an embodiment of the present application, after the distal portion 220 is delivered to a target lesion site, the steering members 42 can be manipulated to pull to the steering traction elements 41, thereby causing the distal portion 220 to bend at a desired angle compatible with the geometry of the lesion, which enables better contact and abutment, improving therapeutic outcomes. Optionally, the steering members 42 may be manipulated to achieve conformal contact and abutment with a lesion in need of ablation by adjusting the bending angle of the distal portion 220 within the range of 0° to 90°. Here, the term "conformal" means that the bending angle is adjusted according to the shape of the lesion so that desirable contact or abutment is achieved between the distal portion 220 and the lesion.

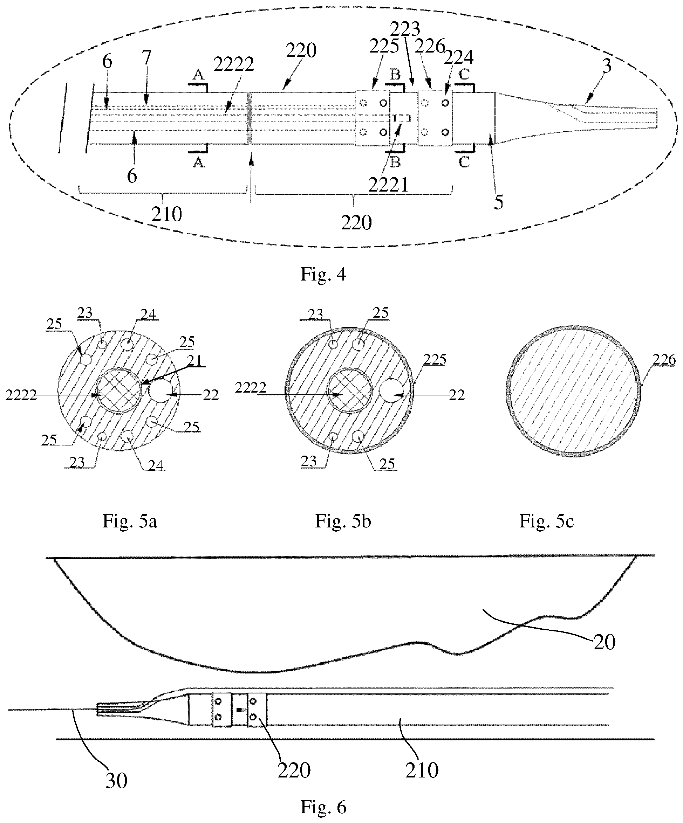

In order to enable the steering traction elements 41 in the steering component 4 to deliver the steering function, in some embodiments, the catheter body 2 defines individual steering channels 25 (see Fig. 5a) for passage of the steering traction elements 41 therethrough. In an alternative embodiment, the steering channels 25 are omitted, and the steering traction elements 41 are instead co-deployed in one or more other channels with other elements. This can reduce the number of required channels, facilitating control of the overall size of the medical catheter 10.

In an exemplary embodiment, there are four steering traction elements 41. As shown in Fig. 5a, in one specific example, four steering channels 25 are provided for the respective steering traction elements 41, four steering channels 25 are provided around the central axis of the catheter body 2 at equal angular intervals of 90°. Each single one of the steering traction elements 41 is arranged in a respective one of the steering channels 25.

Preferably, the distal ends of the steering traction element 41 are arranged proximally with respect to the image-based diagnostic member 222, more precisely, proximally with respect to the imaging probe 2221, as detailed below, in order to avoid interference with the imaging probe 2221, which may affect its imaging performance and quality. However, in practice, the steering traction elements 41 are coupled to the distal portion 220 at locations determined according to the bending angle.

As shown in Figs. 1 and 2, in one embodiment, the proximal end of the proximal portion 210 is coupled to the interface portion 1. The interface portion 1 is adapted for connection with external devices and for input and output of information. The external devices may include an imaging system, an energy output system, a fluid filling device and a driving means. Information that can be input and output through the interface portion 1 includes at least energy. Such energy includes at least energy necessary for imaging, such as light or electrical energy. The interface portion 1 includes a number of interfaces. The number and types of interfaces may depend on the functionality of the distal portion 220.

As shown in Figs. 1 and 2, in one embodiment, a tip 3 is coupled to a distal end of the distal portion 220 by a coupling portion 5. The coupling portion 5 is a solid portion serving to couple the distal portion 220 to the tip 3. The solid coupling portion 5 may couple the distal portion 220 to the tip 3 so as to close and seal a distal end of the distal tubular body. Preferably, the coupling portion 5 is an elastomer, which reduces the risk of the tip 3 causing damage. The coupling portion 5 may be made of polyurethane or silicone, and the diameter of the coupling portion 5 is equal to that of the catheter body 2. An axial length of the coupling portion 5 should not be excessively long or excessively short. If it is too long, the pushing performance of the medical catheter 10 will be degraded. If the length is too short, it may be not able to provide sufficient protection to the tip 3. Preferably, the axial length of the coupling portion 5 ranges from 1 mm to 10 mm.

The tip 3 is tapered, and is usually a soft, non-invasive structure, which allows the tip to cause less damage to blood vessels or tissue. Preferably, the tip 3 defines a guidewire lumen 31, through which a guidewire can be passed to enable rapid exchange. Providing the guidewire lumen 31 in the tip, which is a leading portion of the medical catheter 10, can facilitate exchange and operation of the medical catheter 10 while not expanding its overall size. A diameter of the guidewire lumen 31 may be determined taking into account a diameter of the guidewire. For example, the diameter of the guidewire lumen 31 may be 0.1 mm to 2 mm. The size of the tip 3 should not be excessively large. Otherwise, it may be difficult to cross a stenotic lesion. For this reason, the tip 3 is relatively small in size. Additionally, the tip 3 should not be axially too long, because it will be otherwise sharp and tend to cause damage to blood vessels or tissue. On the other hand, if the length of the tip 3 is too short, its crossability may be degraded. Optionally, the axial length of the tip 3 may be 5.0 mm to 50 mm, preferably 20 mm.

In one embodiment, as shown in Fig. 2, the image-based diagnostic member 222 includes an imaging probe 2221, the imaging probe 2221 is adapted for transfer of energy necessary for imaging and collected signals through an imaging transmission structure 2222. Optionally, one end of the imaging transmission structure 2222 may be coupled to the imaging probe 2221, and the other end thereof may be passed through the distal portion 220 and the proximal portion 210 and coupled to the interface portion 1. Examples of the imaging probe 2221 may include, but are not limited to, micro-lenses (focusing optics), an ultrasonic probes and optical reflectors. The imaging probe 2221 may be implemented as at least one of a micro-lens, an ultrasonic probe and an optical reflector. That is, the image-based diagnostic member 222 may utilize a single imaging approach, or a combination of two or more imaging approaches.

In one embodiment, the image-based diagnostic member 222 employs OCT imaging, in which an imaging optical fiber with ultra-low propagation losses and an extremely small diameter (e.g., 200 µm) is used as a light guide medium, and a micro-lens is provided at a distal end of the imaging optical fiber as the imaging probe 2221. The micro-lens may be a focusing optic optionally implemented as a spherical or gradient refractive index (GRIN) lens. Since the optical fiber is, per se, a fragile glass fiber, it is very easy to break if not protected during use. In order to avoid this, the imaging optical fiber is entirely cladded with a protective sleeve to form an optical fiber cable. This can prevent mechanical damage to the optical fiber and the distal focusing optic during movement and impart to them increased resistance to tensile and flexing forces. The protective sleeve may be selected as a transparent tube.

As shown in Fig. 4, in conjunction with Figs. 5a and 5b, the catheter body 2 further includes an imaging channel 21 for receiving the imaging transmission structure 2222 therein. The imaging channel 21 extends along a central axis of the catheter body 2, preferably, the central axis of the imaging channel 21 coincides with the central axis of the catheter body 2. That is, the imaging channel 21 is located at the center of the medical catheter 10. This facilitates arrangement of channels of other functions around the imaging channel 21, enabling the internal space of the catheter body 2 to be efficiently utilized to ensure sufficient strength of the catheter body 2 without expanding the catheter's outer diameter. This allows easier access of the catheter to a narrow blood vessel in need of therapeutic intervention.

As shown in Figs. 2 and 4, the distal portion 220 further includes an imaging window 223, and the imaging probe 2221 is disposed at the imaging window 223. The imaging window 223 is preferred to be transparent. The imaging window 223 facilitates light emission and reception of the imaging probe 2221. The imaging window 223 may have the same axial length as the distal tubular body. That is, the entire distal tubular body may be transparent. Alternatively, the axial length of the distal tubular body may be greater than the axial length of the imaging window 223. That is, the distal tubular body may be partially transparent. Optionally, the axial length of the imaging window 223 may range from 2 mm to 100 mm. The axial length of the imaging window 223 may be measured from a proximal end of the coupling portion 5 towards the catheter body 2. The transparent portion of the distal tubular body may be made of transparent nylon or the like. Generally, the proximal portion 210 is made of an opaque material, such as polyamide. It is to be noted that the imaging window 223 may be omitted if optical imaging is not adopted.

In one embodiment, the image-based diagnostic member 222 employs optical imaging. In this case, the imaging transmission structure 2222 includes an imaging optical fiber, and the imaging probe 2221 is an optical probe. Preferably, the imaging transmission structure 2222 further includes a protective tube and a torsion spring. The protective tube is disposed over the imaging optical fiber, the torsion spring is provided between the protective tube and the imaging optical fiber. One end of the imaging optical fiber is coupled to the imaging probe 2221, and the other end thereof is coupled to the interface portion 1. The torsion spring is provided to facilitate transmission of torques for driving movement of the imaging optical fiber and the imaging probe 2221 at the distal end of the imaging optical fiber.

In one embodiment, as shown in Fig. 2, the interface portion 1 includes an imaging interface 12 and a mechanical-power transmission interface 14. The other end of the imaging optical fiber is coupled to both the mechanical-power transmission interface 14 and the imaging interface 12 to enable the image-based diagnostic member 222 to be driven by an external driving means to rotate circumferentially and/or translate axially with respect to the catheter body 2. The mechanical-power transmission interface 14 may be integrated with the imaging interface 12, or separate therefrom. Through the mechanical-power transmission interface 14, the driving means can drive movement and rotation of the imaging transmission structure 2222 and the imaging probe 2221. The driving means may be selected as a motor.

In one embodiment, the therapeutic component 211 includes an energy output means for outputting therapeutic energy. The energy output means is able to output at least one of RF radiation, ultrasound waves, laser light and a cryofluid as the therapeutic energy. The energy output means may be implemented as at least one of electrodes for outputting RF radiation, an ultrasonic transducer for producing ultrasound waves, a laser focusing lens for outputting laser light and a cryofluid passage sealed within the medical catheter 10 and configured to transmit energy necessary for cryoablation. That is, the energy output means can output a single type of therapeutic energy, or a combination of two or more types of therapeutic energy.

In one embodiment, the therapeutic member 221 includes a therapeutic agent output means for releasing a therapeutic agent to the target site. The therapeutic agent output means may be of any suitable structure. For example, it may include at least one of a drug-containing coating and a drug-releasing structure. The drug-releasing structure includes a drug-delivery reservoir and/or drug-delivery microneedles. As would be appreciated, the drug-delivery microneedles may be arranged on the outer circumferential surface of the distal tubular body, and a drug may be either stored in advance, or delivered through a drug-delivery channel. The drug-containing coating may be provided on the outer circumferential surface of the distal tubular body. The drug-delivery reservoir may be provided in the outer circumferential surface of the distal tubular body so as to extend through a wall thereof and come into communication with the drug-delivery channel. The therapeutic agent output means may utilize one or more of the aforementioned mechanisms. For example, it may include both the drug-containing coating and the drug-releasing structure. It is noted that either or both of the energy output means and the therapeutic agent output means may be provided.

When the therapeutic member 221 includes the energy output means, the distal portion 220 is preferred to further include temperature-measuring elements (not shown) for measuring a surface temperature of tissue of interest (e.g., tissue in need of ablation) in an energy-based therapeutic procedure, ensuring an appropriate level of output energy. The temperature-measuring elements are able to acquire more accurate temperature information of a lesion, which helps improve therapeutic outcomes. The temperature-measuring elements may be implemented as any suitable mechanisms, such as thermocouples, thermistors or lenses capable of collecting thermal signals. The temperature-measuring elements may be implemented as at least one of thermocouples, thermistors and lenses capable of collecting thermal signals. In one embodiment of the present application, the energy output means includes electrodes, on which the temperature-measuring elements are disposed to directly monitor surface temperatures of the electrodes, and the surface temperature of the tissue of interest can be determined based on the electrodes' surface temperatures.

In a modified embodiment, as shown in Figs. 5a and 5b, the catheter body 2 further defines a temperature-control fluid channel 22 extending along the axis thereof, the temperature-control fluid channel 22 is separate from the imaging channel 21.

As shown in Fig. 4, in one embodiment, the distal portion 220 further includes temperature-control fluid output holes 224, the temperature-control fluid output holes 224 is provided in the electrodes. The distal end of the temperature-control fluid channel 22 is connected to the temperature-control fluid output holes 224, and the proximal end of the temperature-control fluid channel extends to the proximal end of the catheter body 2. For example, the proximal end of the temperature-control fluid channel 22 may be coupled to the interface portion 1. The temperature-control fluid channel 22 is configured for transfer of a temperature-control fluid, and the temperature-control fluid output holes 224 are configured for release of the temperature-control fluid at a given temperature (e.g., hot or cold) to tissue of interest (e.g., tissue in need of ablation), which can reduce damage to the tissue due to excessive heat or cold. Thus, in an energy-based therapeutic procedure, the fluid can be delivered at a cold or hot temperature to the tissue of interest through the temperature-control fluid channel 22 to maintain temperatures of both the medical catheter 10 and a surface of the tissue, with which the medical catheter 10 is brought into contact, within the normal body temperature range, providing protection to unintended sites and increasing safety of the therapeutic procedure. An excessively large diameter of the temperature-control fluid output holes 224 may affect the overall shape of the electrodes and hence the ablation efficacy. On the other hand, if the diameter of the temperature-control fluid output holes 224 is too small, the fluid may be unable to flow out thereof depending on its viscosity. Preferably, the temperature-control fluid output holes 224 are micro-holes with a diameter of 50 µm to 200 µm. The micro-holes are provided to reduce the influence of output of the temperature-control fluid on the electrodes. The temperature of the temperature-control fluid may be adjusted, as required, for example, within the range of 15-30 °C. The temperature of the temperature-control fluid and its output energy and power may be both adjustable to completely protect the endothelium from the ablation energy.

In one embodiment, as shown in Figs. 5a and 5b, the catheter body 2 further defines temperature-control lead channels 23 and electrode lead channels 24, which both extend along the axis thereof and separate from each other. Both the temperature-control lead channels 23 and the electrode lead channels 24 are arranged around the imaging channel 21. In one embodiment, the imaging channel 21, the temperature-control fluid channel 22, the temperature-control lead channels 23, the electrode lead channels 24 and the steering channels 25 are separate from one another without mutual interference or influence. The steering channels 25, the temperature-control lead channels 23, the electrode lead channels 24 and the temperature-control fluid channel 22 may be arranged on a single circle. That is, these channels may have their centers located on a single circle and be arranged around the imaging channel 21. It is also possible to arrange the steering channels 25, the temperature-control lead channels 23, the electrode lead channels 24 and the temperature-control fluid channel 22 on different circles, without limiting the present invention.

The temperature-measuring elements are coupled to the distal end of the temperature-control leads 6, and the proximal end of the temperature-control leads 6 extends through the temperature-control lead channels 23 to the proximal end of the catheter body 2. For example, they may be coupled to the interface portion 1, particularly to the electrical interface 13 in the interface portion 1. The electrodes are coupled to the distal end of the electrode leads 7, and the proximal end of the electrode leads 7 extends through the electrode lead channels 24 to the proximal end of the catheter body 2. For example, they may be coupled to the interface portion 1, particularly to the electrical interface 13.

When the therapeutic member 221 includes the therapeutic agent output means, the catheter body 2 further defines a drug-delivery channel (not shown) extending along the axis thereof and arranged externally to the imaging channel 21. The distal end of the drug-delivery channel is connected to the drug-releasing structure and the proximal end of the drug-delivery channel is connected to the interface portion 1. In this case, the drug-delivery channel is configured for transfer of a drug, and the drug-releasing structure includes a drug-delivery reservoir and/or drug-delivery microneedles and is configured to release the drug to the tissue of interest.

The present application is also not limited to any particular form of the channels in the catheter body 2. In one implementation, the catheter body 2 may itself be a multi-lumen tube, and the channels may be provided by respective lumens of the multi-lumen tube. Alternatively, the catheter body 2 may be a single-lumen tube, and the channels may be defined by a number of separators arranged within the catheter body 2. Still alternatively, the catheter body 2 may be a single-lumen tube, and the channels may be provided by respective smaller tubes wrapped in the single-lumen tube. In each case, the catheter body 2 should provide sufficiently strong support to the individual channels. The channels in the catheter body 2 may be spaced apart from one another, or arranged in close neighborhood and optionally securely connected together.

In a specific example, as shown in Fig. 2, the interface portion 1 includes a fluidic interface 11 for filling of a fluid, an imaging interface 12, an electrical interface 13 for transmission of electrical signals (e.g., electrical currents) and a mechanical-power transmission interface 14 for transmission of mechanical power. The fluidic interface 11 is adapted for connection to a fluid filling device for filling a temperature-control fluid into the medical catheter 10, which is typically normal solution, such as a cold saline solution. The imaging interface 12 is adapted for connection to an imaging system capable of outputting energy necessary for imaging to the medical catheter 10, receiving collected signals from the medical catheter 10, deriving images from the signals, and displaying the images. The electrical interface 13 is adapted for connection to an energy output system, such as an RF system, for outputting therapeutic energy, such as RF energy, to the medical catheter 10. The electrical interface 13 may also be adapted to output electrical energy necessary for temperature monitoring to the medical catheter 10. The electrical interface 13 may also be connected to an external control system for receiving temperature signals from the medical catheter 10. The mechanical-power transmission interface 14 is adapted for connection to a driving means for driving the image-based diagnostic member 222 to rotate and translate to adjust the imaging probe 2221 to a position and orientation in alignment with a target site and suitable for imaging thereof.

In one embodiment, the energy output means includes an electrode for outputting RF energy necessary for RF ablation. Either one or more electrodes may be included. A plurality of electrodes are included, which are spaced axially and/or circumferentially with respect to the catheter body 2. The electrodes may be annular or not. In case of annular electrodes, the catheter is suitable for ablation of concentric, diffuse plaques. In case of non-annular electrodes, the catheter is suitable for ablation of eccentric plaques. Preferably, the proposed medical catheter 10 of this embodiment includes both annular and non-annular electrodes, the proposed medical catheter 10 is therefore suitable for therapeutic treatment of lesions at various locations, meeting different clinical therapeutic needs.

The electrodes may be in the shape of rings or strips, and may be made as sheets or meshes. Without limitation, the electrodes may be radiopaque or not. The present application is not limited to any particular material, from which the electrodes are fabricated. For example, they may be RF electrodes made of a platinum-iridium alloy, platinum, copper, iron, stainless steel or the like. In addition, each electrode must be separated from any other adjacent electrode by a prescribed insulating distance, which should not be excessively small or excessively large. Pulsed electric fields are generated between positive and negative electrodes as electrode signals. If the inter-electrode insulating distance is too small, electric sparks and low-temperature plasmas will tend to occur. If the insulating distance is too large, the strength of the generated electric fields will be adversely affected. For these reasons, the inter-electrode insulating distance should not be arbitrary. Instead, it must ensure that the energy and strength of the generated electric fields will not cause ionization, thus guaranteeing the safety of energy delivered to a lesion. In one embodiment, the inter-electrode insulating distance is between 1 mm and 5 mm, which allows accurate ablation range control while preventing the occurrence of discharge due to an inadequate spacing between any two electrodes. The electrodes may have any required dimensions. In one embodiment, the electrodes may have a width of 2 mm to 10 mm measured along an axis of the medical catheter 10 and a thickness of 0.05 mm to 0.5 mm measured along a radial direction of the medical catheter 10. It would be appreciated that the dimensions of the electrodes should depend on the size of a lesion in need of treatment. In general, larger electrodes mean a greater ablation range. Given the fact that most plaques have a size of around 1 mm, therapeutic needs can be substantially satisfied by configuring the axial width of the electrodes in the range of 2 mm to 10 mm.

As shown in Figs. 3 and 4, in conjunction with Figs. 5a to 5c, there may be two electrodes, for example, which are a proximal electrode 225 and a distal electrode 226. The temperature-control fluid output holes 224 may be provided in the proximal electrode 225 and/or the distal electrode 226, preferably in the proximal electrode 225. In this case, through the temperature-control fluid output holes 224 in the proximal electrode 225, the temperature-control fluid may be released until it fills the entire space between the proximal electrode 225 and the distal electrode 226, where ablation is to be carried out. In the illustrated embodiment, the proximal electrode 225 and the distal electrode 226 are both annular and axially spaced apart from each other by a gap, the imaging probe 2221 is disposed between the proximal electrode 225 and the distal electrode 226. The gap between the proximal electrode 225 and the distal electrode 226 represents the aforementioned space, in which RF ablation is to be performed on a lesion and monitored by the imaging probe 2221 for therapeutic efficacy. In one embodiment, the steering traction elements 41 are distally coupled to the distal tubular body at locations corresponding to a proximal end of the proximal electrode 225.

Each of the electrodes is coupled to a respective one of the electrode leads 7. In particular, each electrode lead 7 may be welded to an inner surface of the respective electrode. The distal end of each electrode lead 7 is passed through a tube and coupled to the inner surface of the respective electrode. The electrode leads 7 may be made of a material, as required. For example, the material of the electrode leads 7 may be selected as one with a suitable resistance value per unit length. The electrode leads 7 and the temperature-control leads 6 may be coupled to the same electrical interface 13, or to two separate electrical interfaces 13.

The medical catheter 10 has an outer diameter, which should be determined taking into account diameters of lumens that it is intended for use with. Optionally, the outer diameter of the medical catheter 10 may range from 1.0 mm to 10.0 mm, which makes it suitable for use with any blood vessel in the human body. The outer diameter of the medical catheter 10 may not exceed 2 mm, which makes it suitable for ablation of smaller plaques. It is to be understood that the outer diameter of the medical catheter 10 is essentially defined by outer diameters of the catheter body 2, the coupling portion 5 and the tip 3. Typically, the outer diameter of the catheter body 2 is equal to that of the coupling portion 5, and the outer diameter of the tip 3 at its proximal end is equal to the outer diameter of the catheter body 2.

In a specific application, the proposed medical catheter 10 is used to treat a coronary artery. In this case, the outer diameter of the catheter body 2 is 1.0 mm to 3.0 mm, preferably 1.8 mm to 2.0 mm. If the outer diameter of the catheter body 2 were larger than 3.0 mm, the catheter would be difficult to use for coronary intervention due to bulkiness. If the outer diameter of the catheter body 2 were less than 1.0 mm, it would be difficult to integrate the aforementioned channels therein, and the manufacturing would encounter greater difficulties. The catheter body 2 may have a wall thickness of 0.1 mm to 0.5 mm, which ensures both sufficient overall strength and good compliance of the medical catheter 10. Further, the imaging channel 21 has a diameter not exceeding 1.0 mm, and the temperature-control fluid channel 22 has a diameter not exceeding 0.5 mm. Each electrode lead channel 24 has a diameter of 0.1 mm to 0.5 mm, and each temperature-control lead channel 23 has a diameter of 0.1 mm to 0.5 mm. Such diameters enable adequate accommodation of the channels with reduced interference therebetween, allowing them to deliver their intended functions.

In a specific embodiment, as shown in Figs. 3 to 4 and 5a to 5c, the medical catheter 10 is used as an ablation catheter and employs optical coherence tomography (OCT) and RF ablation to conformally treat an atherosclerotic plaque. In this embodiment, the outer diameter of the catheter body 2 is 2.0 mm, and the catheter body 2 defines one imaging channel 21, one temperature-control fluid channel 22, two temperature-control lead channels 23, two electrode lead channels 24 and four steering channels 25. The temperature-measuring elements are implemented as thermocouples, and two annular electrodes are provided, which are spaced apart from each other along the axis of the catheter body 2. Both annular electrodes are made of a platinum-iridium alloy and have a thickness of 0.1 mm and an axial width of 2.0 mm. The transparent imaging window 223 is arranged between the two annular electrodes, and the transparent imaging window 223 is totally 50 mm long. The imaging probe 2221 is disposed between the two annular electrodes, to each of which an electrode lead 7 made of platinum-iridium alloy and a temperature-control lead 6 made of a copper-nickel alloy are welded. The two temperature-control leads 6 and the two electrode leads 7 are arranged around the imaging channel 21 in symmetry with respect thereto. Each annular electrode defines 12 temperature-control fluid output holes 224 having a diameter of 90 µm, which are evenly arranged circumferentially with respect to the electrode. Four steering traction elements 41 are received in the respective steering channels 25, the four steering traction elements 41 are coupled to respective steering members 42 proximally and to the distal tubular body distally at locations corresponding to the proximal end of the proximal electrode 225. The temperature-control fluid channel 22, the two temperature-control lead channels 23, the two electrode lead channels 24 and the four steering channels 25 are arranged around the imaging channel 21, the imaging channel 21 is disposed at the center of the medical catheter 10. The temperature-control fluid channel 22, the two temperature-control lead channels 23, the two electrode lead channels 24 and the four steering channels 25 are arranged on a single circle, in order to allow the catheter to have a reduced overall outer diameter. The diameter of the imaging channel 21 is 0.5 mm, and the diameter of the temperature-control fluid channel 22 is 0.3 mm. The two temperature-control lead channels 23 are arranged in symmetry with respect to the imaging channel 21, and the two electrode lead channels 24 are also arranged in symmetry with respect to the imaging channel 21. With this arrangement, light, electricity and heat can be transmitted in a compatible manner with reduced mutual influence and interference.

In a surgical procedure, the ablation catheter is advanced over the guidewire running through the guidewire lumen 31 in the tip 3 into a blood vessel. The tip 3 adopts a rapid exchange design, and providing the guidewire lumen 31 in the tip, which is a leading portion of the medical catheter 10, allows for rapid exchange of interventional instruments and enables the catheter to have a reduced overall size. The guidewire lumen 31 has a diameter of 0.5 mm, and the tip 3 has an axial length of 30 mm. The tip 3 is coupled, for example, glued, to the distal portion 220 by the coupling portion 5, which is made of polyurethane. The coupling portion 5 has an outer diameter of 2.0 mm and an axial length of 20 mm. The imaging interface 12 and the mechanical-power transmission interface 14 are integrally formed and together coupled to an imaging system. The image-based diagnostic member 222 utilizes the imaging optical fiber in combination with a GRIN lens to emit laser light and collect optical signals reflected from the blood vessel to monitor the RF ablation procedure. The imaging probe 2221 is implemented as a spherical or gradient refractive index (GRIN) lens disposed between the two annular electrodes to receive and emit a light beam through the imaging window 223. The imaging optical fiber is axially translated and rotated in the imaging channel 21 by a driving means, concurrently with the GRIN lens emitting and collecting optical signals. During ablation, a cold saline solution flows from a fluid filling device through the fluidic interface 11 into the temperature-control fluid channel 22. It then further flows through the temperature-control fluid output holes 224 in the electrodes to a lesion in need of RF ablation, thereby cooling down the lesion while not affecting endothelial cells. The cold saline solution can be supplied at an adjustable flow rate, satisfying various different cooling requirements.

Use of the proposed medical catheter 10 may include the steps as follows:
1) Connect the imaging interface 12 to an imaging system, the electrical interface 13 to an energy output system, the fluidic interface 11 to a fluid filling device, and the mechanical-power transmission interface 14 to the driving means.
2) Deliver the medical catheter 10 over the guidewire into a diseased blood vessel segment and then activate the imaging system. Once activated, the imaging system captures images of the diseased region, from which locations and compositions of the lesions are identified.
3) After the image-based diagnosis, advance the distal portion 220 to the location of an identified lesion and then bend the distal portion 220 in a desired direction at a desired angle relative to the proximal portion 210 by manipulating the steering component 4 so that the distal portion 220 is brought into close contact and abutment with the lesion.
4) After close contact and abutment is attained between the distal portion 220 and the lesion, activate the energy output system, which then delivers therapeutic energy and/or a therapeutic agent toward the lesion. In this process, the imaging probe 2221 continuously monitors, through the imaging window 223, efficacy at a specified location or delivery of the therapeutic agent.
5) After the therapeutic treatment of the current lesion is complete, release the distal portion 220, which responsively recovers the original shape of the distal portion 220. Afterwards, the medical catheter 10 is advanced to the next lesion, the above therapeutic process is repeated.
6) After therapeutic treatment of the entire diseased region is completed, evaluate the therapeutic efficacy by scanning the whole blood vessel segment.
7) Finally, withdraw the medical catheter 10 over the guidewire. This ends the therapeutic procedure.

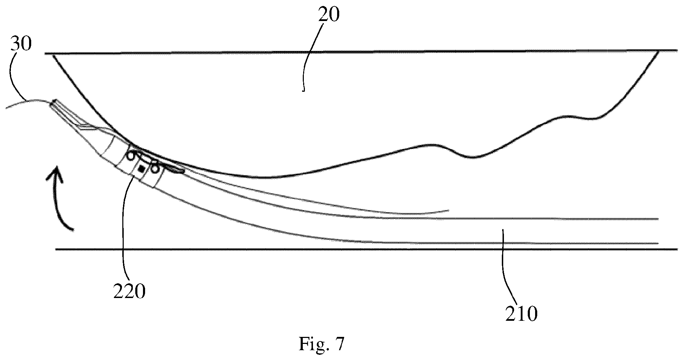

More specifically, referring to Figs. 6 and 7, before the procedure begins, the interfaces in the interface portion 1 are respectively coupled to the corresponding imaging system, RF system and fluid filling device. The medical catheter 10 is then advanced into the blood vessel over the guidewire 30 that is passed through the guidewire lumen 31 in the tip 3, and the imaging optical fiber and the imaging probe 2221 are translated and rotated in the imaging channel 21 by mechanical forces, concurrently with the imaging probe 2221 emitting laser light and collecting optical signals reflected from the blood vessel. Images of the blood vessel are captured by the imaging system using OCT, and plaques are then analyzed for their morphology and composition based on the images. Subsequently, according to the results of the analysis, the electrodes are aligned with a fibrous plaque 20 to be ablated. The steering members 42 are manipulated to adjusting the bending angle of the distal portion 220, thereby bringing it into close contact and abutment with the fibrous plaque 20, as shown in Fig. 7. The RF system is then activated to apply RF currents through the electrical interface 13 to generate resistive heat to ablate the plaque in the blood vessel. In this process, the imaging optical fiber continuously rotates and collects image signals in real time to monitor progress of the ablation, and the thermocouples on the surfaces of the electrodes carry out real-time temperature monitoring. At the same time, a cold saline solution is continuously supplied from the temperature-control fluid output holes 224 to flood the surfaces of the electrodes and the tissue being treated, thus conditioning the temperature and reducing possible damage to endothelial cells. After the ablation of the current plaque is completed, the distal portion 220 is released (see Fig. 6), and the medical catheter 10 is moved to the next lesion, followed by repeating the above ablation process thereon. After treatment of the last lesion is completed, efficacy evaluation is carried out on the whole blood vessel based on OCT imaging. At last, the catheter is withdrawn, ending the procedure.

It is particularly noted that the ablation catheter is not limited to being an RF ablation catheter and may be alternatively implemented as a cryoablation catheter. In the latter case, the tubular body may define a cryofluid channel therein, and a cryofluid may be jetted from a distal outlet port of the cryofluid passage onto an inner surface of a balloon possibly disposed over the distal portion 220. It will be recognized that, so far, there has been no technique for RF ablation of atherosclerotic plaques under the guidance of intravascular imaging and temperature control. The proposed catheter can be made with an outer diameter not greater than 2 mm, making it suitable for thermal ablation of smaller plaques. Combining RF ablation with imaging and temperature monitoring enables more accurate RF ablation with better efficacy. In particular, steering capabilities are further added to the catheter with an outer diameter less than 10 mm, especially of 2 mm or less, which allow conformal contact of the therapeutic member with a lesion in need of treatment, and hence compatibility of the therapeutic member with the geometry of the lesion. This enables more accurate targeting of treatment, which results in improved therapeutic outcomes.

The medical catheter proposed herein can employ one or more approaches to diagnose and therapeutically treat vascular, cardiac and other diseases. For example, OCT imaging can be used prior to a therapeutic procedure to carry out therapeutic planning. Moreover, OCT imaging and temperature monitoring can be used to provide real-time efficacy feedback during the therapeutic procedure, and to evaluate the overall therapeutic efficacy after the therapeutic procedure is completed, resulting in effective improvements in therapeutic efficacy. In addition, the catheter can be manufactured as a modular component system, in which various functional modules can be incorporated. This overcomes the challenge of deployment and functional delivery in limited spaces and provides for a multi-functional catheter with both diagnostic and therapeutic capabilities, which is expected to provide effectively improved efficacy in clinical applications.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical catheter, comprising a catheter body and a steering component, the catheter body comprising a proximal portion and a distal portion at a distal end of the catheter body, the distal portion configured for image-based monitoring and for release of therapeutic energy and/or a therapeutic agent, the distal portion connected to the steering component and configured to bend relative to the proximal portion under the control of the steering component.

2. The medical catheter according to claim 1, wherein the distal portion comprises a distal tubular body and the proximal portion comprises a proximal tubular body, wherein hardness of the distal tubular body is less than hardness of the proximal tubular body.

3. The medical catheter according to claim 1 or 2, wherein the steering component comprises a steering traction element and a steering member, the steering member disposed at a proximal end of the catheter body, a distal end of the steering traction element connected to the distal portion, a proximal end of the steering traction element passed through the catheter body and then connected to the steering member, the steering traction element configured to be driven by the steering member to control the bending of the distal portion.

4. The medical catheter according to claim 3, wherein a plurality of steering traction elements are provided, which are equidistantly arranged circumferentially around a central axis of the catheter body.

5. The medical catheter according to claim 4, wherein each of the steering traction elements is connected to at least one of steering members, different steering traction elements being connected to different steering members.

6. The medical catheter according to claim 3, further comprising an interface portion, wherein a proximal end of the proximal portion is connected to the interface portion, and the steering member is movably disposed on the interface portion.

7. The medical catheter according to claim 3, wherein the catheter body further comprises a steering channel extending along an axis thereof, wherein the steering traction element is passed through the steering channel.

8. The medical catheter according to claim 1 or 2, wherein the steering component comprises a magnetically responsive deformable component and/or a photo-deformable component, the magnetically responsive deformable component made of a magnetically responsive material and deformable under the action of a magnetic field, the photo-deformable component made of a photo-responsive material and photo-deformable when absorbing light energy.

9. The medical catheter according to claim 1 or 2, further comprising an interface portion, wherein a proximal end of the proximal portion is connected to the interface portion, and the interface portion is configured for connection with an external device and for input and output of information.

10. The medical catheter according to claim 1 or 2, wherein the distal portion comprises an image-based diagnostic member and a therapeutic member, the image-based diagnostic member configured for the image-based monitoring, the therapeutic member configured for the release of the therapeutic energy and/or the therapeutic agent,
the image-based diagnostic member comprising an imaging probe, wherein the catheter body further comprises an imaging channel extending along an axis thereof, an imaging transmission structure connected to the imaging probe is disposed in the imaging channel, and a central axis of which coincides with the central axis of the catheter body.

11. The medical catheter according to claim 10, wherein the distal portion further comprises an imaging window, and wherein the imaging probe is provided at the imaging window.

12. The medical catheter according to claim 10, wherein the imaging probe is an optical probe, wherein the imaging transmission structure comprises an imaging optical fiber, a protective tube and a torsion spring, the protective tube disposed over the imaging optical fiber, the torsion spring disposed between the protective tube and the imaging optical fiber, one end of the imaging optical fiber connected to the imaging probe, the other end of the imaging optical fiber extending through the imaging channel to a proximal end of the catheter body.

13. The medical catheter according to claim 12, further comprising an interface portion, wherein a proximal end of the proximal portion is connected to the interface portion; the interface portion comprises an imaging interface and a mechanical-power transmission interface; the other end of the imaging optical fiber is connected to the mechanical-power transmission interface and the imaging interface; and the image-based diagnostic member is configured to be driven by a driving member to rotate circumferentially and/or translate axially with respect to the catheter body.

14. The medical catheter according to claim 10, wherein the therapeutic member comprises an energy output component capable of outputting at least one of radio frequency (RF) radiation, ultrasound waves, laser light and a cryofluid as the therapeutic energy, and/or the therapeutic member comprises a therapeutic agent output component, the therapeutic agent output component comprising at least one of a drug-containing coating and a drug-releasing structure, the drug-releasing structure comprising a drug-delivery reservoir and/or drug-delivery microneedles.

15. The medical catheter according to claim 14, wherein the therapeutic member comprises the energy output component, wherein the energy output component comprises an electrode, and the distal portion further comprises a temperature-measuring element disposed on the electrode and configured to measure a surface temperature of the electrode.

16. The medical catheter according to claim 15, wherein the catheter body further comprises a temperature-control fluid channel extending along the axis thereof, wherein the distal portion further comprises temperature-control fluid output holes provided in the electrode, and a distal end of the temperature-control fluid channel is connected to the temperature-control fluid output holes and configured for transfer of a temperature-control fluid.

17. The medical catheter according to claim 15, wherein the catheter body further comprises a temperature-control lead channel and an electrode lead channel, both extending along the axis thereof, wherein the temperature-measuring element is connected to a distal end of a temperature-control lead; a proximal end of the temperature-control lead extends through the temperature-control lead channel to the proximal end of the catheter body; the electrode is connected to a distal end of an electrode lead; and a proximal end of the electrode lead extends through the electrode lead channel to the proximal end of the catheter body.

18. The medical catheter according to claim 14, wherein the energy output component comprises a plurality of electrodes for outputting RF radiation, the plurality of electrodes being spaced axially along and/or circumferentially around the catheter body.

19. The medical catheter according to claim 1 or 2, wherein the distal end of the distal portion is connected to a tip through an elastic coupling portion, the tip defining a guidewire lumen.
